Europäisches Patentamt

(19) European Patent Office

Office européen des brevets

(11) **EP 0 852 260 A1**

(12) **EUROPEAN PATENT APPLICATION**
published in accordance with Art. 158(3) EPC

(43) Date of publication:
08.07.1998 Bulletin 1998/28

(21) Application number: 96917654.4

(22) Date of filing: 12.06.1996

(51) Int. Cl.$^6$: **C12N 9/02**, C12N 1/14,
C11D 3/386, D06L 3/00
// (C12N9/02, C12R1:645)

(86) International application number:
PCT/JP96/01594

(87) International publication number:
WO 97/00948 (09.01.1997 Gazette 1997/03)

(84) Designated Contracting States:
AT BE CH DE DK ES FI FR GB GR IE IT LI NL PT
SE

(30) Priority: 23.06.1995 JP 158017/95
18.12.1995 JP 328777/95
23.01.1996 JP 9297/96

(83) Declaration under Rule 28(4) EPC (expert
solution)

(71) Applicant: NOVO NORDISK A/S
2880 Bagsvaerd (DK)

(72) Inventors:
• ECHIGO, Takashi,
Showa Denko K.K.
Chiba-shi, Chiba 267 (JP)
• OHNO, Ritsuko,
Showa Denko K.K.
Chiba-shi, Chiba 267 (JP)
• FUKUYAMA, Shiro,
Showa Denko K.K.
Chiba-shi, Chiba 267 (JP)

(54) **OXIDASE, MICROORGANISMS PRODUCING THE SAME AND USE OF THE SAME**

(57)     A polyphenol oxidase with the optimum reaction pH in an alkaline region above pH 8 and a method for producing the polyphenol oxidase can be provided. *Myrothecium verrucaria* SD3001 and *Myrothecium roridum* SD3002 effectively produce the polyphenol oxidase. The use of the polyphenol oxidase of the present invention realizes effective processes including treatment of coloring substances, treatment of paper, pulp or fiber, bleaching process, cleaning process or sterilization and inactivation process of microorganisms and viruses.

EP 0 852 260 A1

**Description**

**BACKGROUND OF THE INVENTION**

**Field of the Invention**

The present invention relates to a novel oxidase with the optimum reaction pH in an alkaline region and the utilities of the same. More specifically, the present invention relates to a novel microorganism-produced oxidase with the optimum reaction pH in an alkaline region above pH 8, a microorganism for producing the oxidase, a method for producing the oxidase, and a method for utilizing the oxidase in the field of oxidation process of colorants and polyphenol-containing matters and the field of cleaning and the like.

**Description of the Prior Art**

As microorganism-derived enzymes with polyphenol oxidizing action, conventionally, polyphenol oxidase and laccase generated by filamentous fungus such as *Basidiomycetes* and *Deuteromycotina* have been known. Because conventional polyphenol oxidases have the optimum reaction pHs in an acid to neutral region and therefore exert very low activity in an alkaline region above pH 8, the practical utilities thereof have been restricted.

For example, the utilization of polyphenol oxidases for bleaching is described in WO 91-05839, EP 91 610 032, DE 4008894, Japanese Patent Laid-open No. 64-60693 and the like. However, cleaning procedures such as laundry washing are generally conducted at an alkaline pH; for simultaneous oxidation bleaching in the presence of hydrogen peroxide, in particular, washing procedures are preferably carried out at an alkaline pH for facilitating the bleaching action of hydrogen peroxide. When intending to use polyphenol oxidase for such utilities, therefore, conventional such enzymes with the optimum reaction pHs at an acidic pH cannot be applied practically.

It has been known that natural colorants containing polyphenol in the backbone structure include plant dyes such as flavonoids, xanthones and melanines and lignin, and polyphenol oxidase has an oxidizing action over these natural products. As a reaction substrate, additionally, polyphenol oxidase can utilize dichlorophenol and trichlorophenol with toxicity problems. Therefore, polyphenol oxidase is useful for the disposal of liquid waste containing these natural products and non-natural products. Nevertheless, conventional such enzymes substantially cannot be used at alkaline pHs because these enzymes have the optimum reaction pHs in an acidic- to neutral pH region, which is a factor working to restrict the industrially applicable range of polyphenol oxidase.

Some of polyphenol oxidases derived from animals and plants have been known to have the optimum pHs at a high pH above pH 8 (Comp. Biochem. Physiol., 1992, 102B, 4, 891 - 896: Zhongguo Nongye Huaxue Huizhi, 1991, 29, 2, 177 - 185: Agric. Biol. Chem., 1991, 55, 1, 13 - 17). However, these polyphenol oxidases can hardly be produced from the tissues of animals and plants, inexpensively, in a stable fashion. Hence, polyphenol oxidases being derived from microorganisms and having the optimum reaction pHs in an alkaline region have been desired for industrial application.

SUMMARY OF THE INVENTION

It is an object of the present invention to make screening of a microorganism generating a polyphenol oxidase with the optimum reaction pH in an alkaline region above pH 8 to provide the polyphenol oxidase generated by the microorganism, thereby attaining practically the enzymatic oxidation of polyphenols in an alkaline pH region and making contribution to the enlargement of the applicable field of polyphenol oxidase.

BRIEF DESCRIPTION OF THE DRAWINGS

Fig.1 is a graph depicting the pH profile of the polyphenol oxidase derived from the strain SD3001 of the present invention;

Fig.2 is a graph depicting the temperature profile of the polyphenol oxidase derived from the strain SD3001;

Fig.3 is a graph depicting the temperature stability of the polyphenol oxidase derived from the strain SD3001;

Fig.4 is a graph depicting the pH stability of the polyphenol oxidase derived from the strain SD3001;

Fig.5 is a chart depicting the patterns of the absorbance at 280 nm and the activity value of individual fractions derived from the culture broth of the strain SD3001;

Fig.6 is a chart depicting the absorbance of Fraction No.59 derived from the culture broth of the strain SD3001;

Fig.7 is a view depicting the elution pattern of Fraction No.59 derived from the strain SD3001 by GFC analysis (HPLC);

Fig.8 is graph depicting the pH profile of a mixture of the polyphenol oxidase derived from the strain SD3001 with a commercially available polyphenol oxidase;

Fig.9 is the pH profile of the polyphenol oxidase derived from the strain SD3002 of the present invention;

Fig.10 is the temperature profile of the polyphenol oxidase derived from the strain SD3002;

Fig.11 is a graph depicting the temperature stability of the polyphenol oxidase derived from the strain SD3002;

Fig.12 is a graph depicting the pH stability of the polyphenol oxidase derived from the strain SD3002;

Fig.13 is a chart depicting the patterns of the absorbance at 280 nm and the activity value of individual fractions derived from the culture broth of the strain SD3002; and

Fig.14 is a graph depicting the pH profile of a mixture of the polyphenol oxidase derived from the strain SD3002 with a commercially available polyphenol oxidase.

DETAILED DESCRIPTION OF THE INVENTION

The present inventors have made investigations and screenings of a microorganism producing an exoenzyme catalyzing the oxidation of polyphenol substances at alkaline pHs in a wide variety of microorganisms. The screening thereof has involved much difficulty, but the inventors have found eventually that a bacterial strain belonging to the genus *Myrothecium* among *Deuteromycotina* generates the objective enzyme with the optimum reaction pH in an alkaline region above pH 8 outside the bacteria. Thus, the invention has been achieved.

More specifically, the present invention provides those described below.

1) A polyphenol oxidase with the optimum reaction pH in an alkaline region above pH 8, which is derived from a microorganism.

2) The polyphenol oxidase described above in 1, which is derived from the genus *Myrothecium*.

3) The polyphenol oxidase described above in 1 or 2, having the following properties.

(1) Action
Oxidizing polyphenol.
(2) Optimum reaction pH
Having the optimum reaction pH around pH 8.5 to 9.
(3) Optimum reaction temperature
Having the optimum reaction temperature at about 60°C.
(4) Molecular weight
The molecular weight measured by gel filtration chromatography analysis is about 62,000.
(5) Isoelectric point measured by isoelectric electrophoresis is 4.5 ± 0.5.

4) The polyphenol oxidase described above in 1 to 3, which can be recovered from *Myrothecium verrucaria*.

5) The polyphenol oxidase described above in 1 to 3, which can be recovered from *Myrothecium verrucaria* strain SD3001 (Accession No. FERM P-14955).

6) The polyphenol oxidase described above in 1 or 2, having the following properties.

(1) Action
Oxidizing polyphenol.
(2) Optimum reaction pH
Having the optimum reaction pH around pH 9.
(3) Optimum reaction temperature
Having the optimum reaction temperature around 70°C.
(4) Molecular weight
The molecular weight measured by gel filtration chromatography analysis is about 60,000.
(5) Isoelectric point measured by isoelectric electrophoresis is 4.1 ± 0.5.

7) The polyphenol oxidase described above in 1, 2 or 6, which can be recovered from *Myrothecium roridum*.

8) The polyphenol oxidase described above in 1,2 or 6, which can be recovered from *Myrothecium roridum* strain SD3002 (Accession No. FERM P-15255).

9) A method for treating phenol compounds, alkoxyl group-containing aromatic compounds, halogenated phenol compounds or aromatic amine compounds, comprising making the polyphenol oxidase described above in any one of 1 to 8 act thereon.

10) A method for treating coloring substances, comprising making the polyphenol oxidase described above in any one of 1 to 8 act thereon.

11) A method for treating microorganisms or viruses, comprising making the polyphenol oxidase described above in any one of 1 to 8 act thereon.

12) A method for treating paper, pulp or fiber, comprising making the polyphenol oxidase described above in any one of 1 to 8 act thereon.

13) A method for utilizing the polyphenol oxidase described above in any one of 1 to 8, characteristically using the polyphenol oxidase in combination with a cleaning agent, a detergent or a surfactant.

14) A method for utilizing the polyphenol oxidase described above in any one of 1 to 8, characteristically using the polyphenol oxidase in combination with a substance with peroxidase action.

15) A method for utilizing the polyphenol oxidase described above in any one of 1 to 8, characteristically using the polyphenol oxidase in combination with air, oxygen, ozone, hydrogen peroxide, a hydrogen peroxide precursor, a peracid precursor or peracid singly or in combination of a plurality thereof.

16) A method for utilizing the polyphenol oxidase described above in any one of 1 to 8, characteristically using the polyphenol oxidase in combination with the substrate.

17) A method for producing the polyphenol oxidase described above in any one of 1 to 8, comprising culturing the bacteria of the genus *Myrothecium*.

18) A method for producing the polyphenol oxidase described above in 17, wherein the bacteria of the genus *Myrothecium* belong to *Myrothecium verrucaria*.

19) A method for producing the polyphenol oxidase described above in 17, wherein the bacteria of the genus *Myrothecium* belong to *Myrothecium verrucaria* strain SD3001 (Accession No. FERM P-14955) or a variant thereof.

20) A method for producing the polyphenol oxidase described above in 17, wherein the bacteria of the genus *Myrothecium* belong to *Myrothecium roridum*.

21) A method for producing the polyphenol oxidase described above in 17, wherein the bacteria of the genus *Myrothecium* belong to *Myrothecium roridum* strain SD3002 (Accession No. FERM P-15255) or a variant thereof.

22) *Myrothecium verrucaria* strain SD3001 (Accession No. FERM P-14955).

23) *Myrothecium roridum* strain SD3002 (Accession No. FERM P-15255).

24) A detergent composition containing the polyphenol oxidase described above in any one of 1 to 8.

[Producing bacteria]

The bacterial strain of the genus *Myrothecium* to be used for producing the polyphenol oxidase in accordance with the present invention includes *Myrothecium roridum*, *Myrothecium verrucaria*, *Myrothecium prestonii*, and *Myrothecium leucotrichum*; preference is given to *Myrothecium verrucaria*. and *Myrothecium roridum*; *Myrothecium verrucaria* SD3001 or *Myrothecium roridum* SD3002, in particular, is preferably used.

*Myrothecium verrucaria* SD3001 was deposited as FERM P-14955 at the Life Engineering and Industrial Technology Research Institute, the Agency of Industrial Science and Technology, the Ministry of Health and Welfare, at 1-3, Higashi 1-chome, Tsu-kuba-shi, Ibaraki-ken, Japan on May 29, 1995, which was then internationally deposited as FERM BP-5520 on April 24, 1996. Also, *Myrothecium verrucaria* SD3002 was deposited as FERM P-15255 at the Life Engineering and Industrial Technology Research Institute, the Agency of Industrial Science and Technology, the Ministry of Health and Welfare, at 1-3, Higashi 1-chome, Tsu-kuba-shi, Ibaraki-ken, Japan on October 26, 1996, which was then internationally deposited as FERM BP-5523 on April 24, 1996.

The representative bacterial strains of the genus *Myrothecium*, producing the polyphenol oxidase of the present invention, were morphologically observed on a malt extract agar medium, concerning the color, shape and conidium of the resulting colonies and the structure of the formed conidium. The results are shown below.

| Items | Properties |
|---|---|
| Color of colonial surface | white to yellowish white; black sporodochium observed; |
| Color of colonial back face | white to yellowish white |
| Tissue on colonial surface | slightly woolly |
| Hyphae | colorless; smooth surface width of 1.5 to 3 $\mu$m |
| Phialide | cylindrical shape; 3 to 6 phialides whirled size of 10 - 15 x 1.5 - 2 $\mu$m |
| Conidium | spindle shape to lemon shape size of 6 - 8 x 2 - 3.5 $\mu$m some forming appendages of a fan shape on ends |

The bacterial strain was designated as *Myrothecium verrucaria* SD3001, because some of the conidia form appendages of a fan shape on the ends.

Other representative bacterial strains of the genus *Myrothecium,* producing the polyphenol oxidase of the present invention, were cultured on a potato-carrot agar medium (PCA) at 25°C for 14 days, and the color, shape and conidium of the resulting colonies and the structure of the formed conidium were morphologically observed. The results are shown below.

| Items | Properties |
|---|---|
| Growth rate | Colony diameter of 54 to 56 mm |
| Color of colonial surface | white to yellowish white; black sporodochium observed; |
| Color of colonial back face | white to yellowish white |
| Hyphae | colorless; smooth surface; width of 1.5 to 3 $\mu$m |
| Phialide | cylindrical shape; 3 to 6 phialides whirled size of 12 - 16 x 1.5 - 2.5 $\mu$m |
| Conidium | rod shape to slender elliptical shape; round both ends with cut base; size of 5.5 - 7.5 x 1.5 - 2 $\mu$m |

Based on the outcome of the observation, the bacterial strain was designated as *Myrothecium roridum* SD3002.

[Enzyme preparation]

The polyphenol oxidase of the present invention can be produced by culturing the bacterial strain of the genus *Myrothecium* or a variant thereof. Additionally, the polyphenol oxidase can be prepared by utilizing a bacterium genetically manipulated. More specifically, the polyphenol oxidase can be produced by using an expression vector where a DNA sequence encoding the polyphenol oxidase is inserted in a DNA vector with an origin of replication for replicating the vector in a host organism, together with an appropriate promoter DNA sequence with a function of expressing the enzyme, and an operator DNA sequence and a terminator DNA sequence, thereby transforming a host cell, or by using an expression vector where a DNA sequence encoding the polyphenol oxidase is integrated with the DNA of a host cell, together with an appropriate promoter DNA sequence with a function of expressing the enzyme, and an operator DNA sequence and a terminator DNA sequence, thereby transforming the host cell, comprising culturing the host cell under conditions to potentially express the polyphenol oxidase and further recovering the resulting polyphenol oxidase from the culture.

The polyphenol oxidase of the present invention is satisfactorily produced by protein engineering technology for modifying the DNAs of conventional polyphenol oxidases with the optimum reaction pHs in an acidic region, on the basis of the finding of the amino acid sequence of the polyphenol oxidase produced from the DNA sequence encoding the polyphenol oxidase and the like.

So as to recover a DNA fragment encoding the polyphenol oxidase of the present invention, for example, routine methods can be adopted, including a process of identifying the objective DNA fragment by using as the probe an oligonucleotide synthesized on the basis of the amino acid sequence of the polyphenol oxidase of the present invention or of the amino acid sequence of a known polyphenol oxidase and as the separation source the cDNA or genome library from the bacterial strain of the present invention, or a process of selecting a clone expressing the enzyme activity, or a process of selecting a clone generating a protein reactive with an antibody against the polyphenol oxidase.

For culturing the polyphenol oxidase of the present invention, routine synthetic media and nutrient culture media containing organic carbon sources and organic nitrogen sources can be used. Additionally, $Cu^{2+}$ ion is preferably added as the form of a metal salt at a concentration of 0.001 mM to 10 mM, preferably 0.01 mM to 1 mM. The culturing temperature is 10 to 35°C, preferably 20 to 30°C. Further, the culturing time is appropriately 20 to 150 hours, preferably 40 to 100 hours. The secreted polyphenol oxidase can be recovered from the culture media by well known methods. The recovery procedures include a series of procedures such as cell separation from the culture media by centrifugation or filtration or filter separation and chromatography, for example ion exchange chromatography. Additionally, membrane concentration using ultrafiltration membrane is also effective.

[Enzyme properties]

(1) Enzyme derived from *Myrothecium verrucaria* SD3001

The enzyme derived from *Myrothecium verrucaria* SD3001 as a representative one of the polyphenol oxidase of the present invention has a molecular weight of about 62,000, as determined by gel filtration chromatography and an isoelectric point of 4.5± 0.5 as determined by isoelectric electrophoresis.

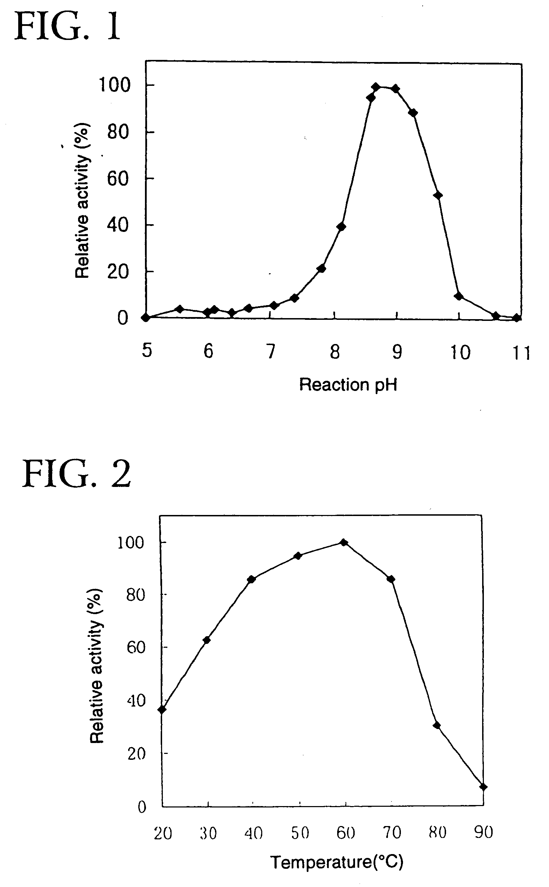

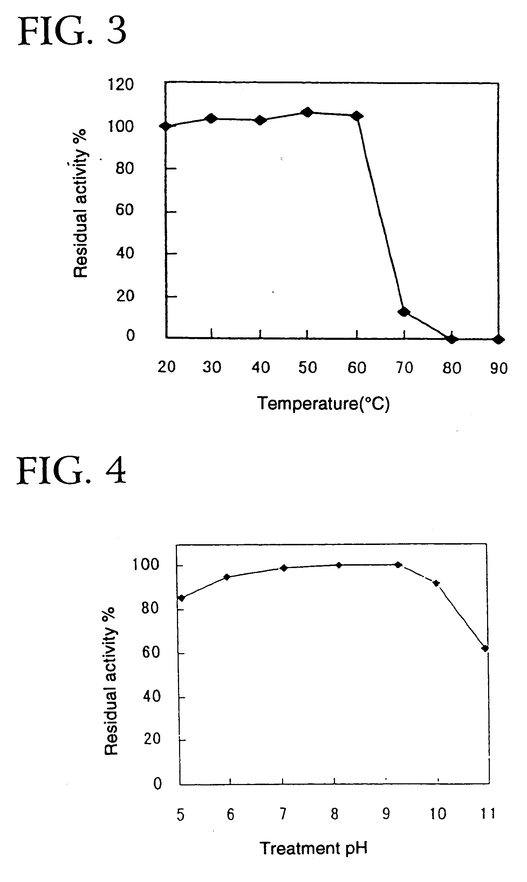

The enzyme can progress polyphenol oxidation reaction in a wide pH range of 5 to 11, and the reaction pH is preferably 7 to 10, more preferably 8 to 9.5, with the optimum pH around pH 8.5 to 9, to characteristically catalyze the oxidation reaction in an alkaline region (Fig.1). Additionally, the optimum temperature is about 60°C (Fig.2). Further, almost 100 % of the activity remained after 30-min heating processes at various given temperatures, for example at 60°C (Fig.3). Still furthermore, the residual activity after 30-min processes in buffer solutions of various pHs at 30°C were stable in a wide pH range (Fig.4). These results verify the oxidation reaction of the enzyme derived from *Myrothecium verrucaria* SD3001 in various solutions at medium to low temperature in a wide pH range from neutrality to alkalinity.

(2) Enzyme derived from *Myrothecium roridum* SD3002

The enzyme derived from *Myrothecium roridum* SD3002 as a representative one of the polyphenol oxidase of the present invention has a molecular weight of about 60,000, as measured by gel filtration chromatography and an isoelectric point of 4.1 ± 0.5, as measured by isoelectric electrophoresis.

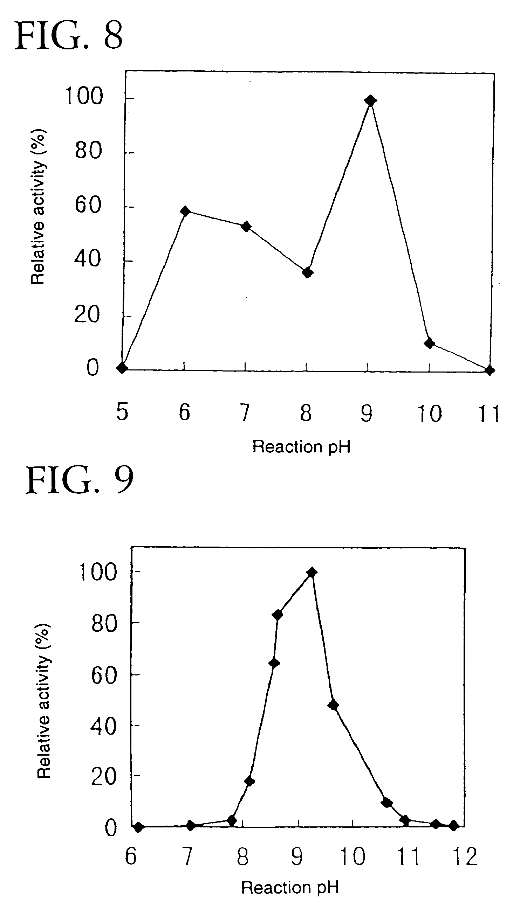

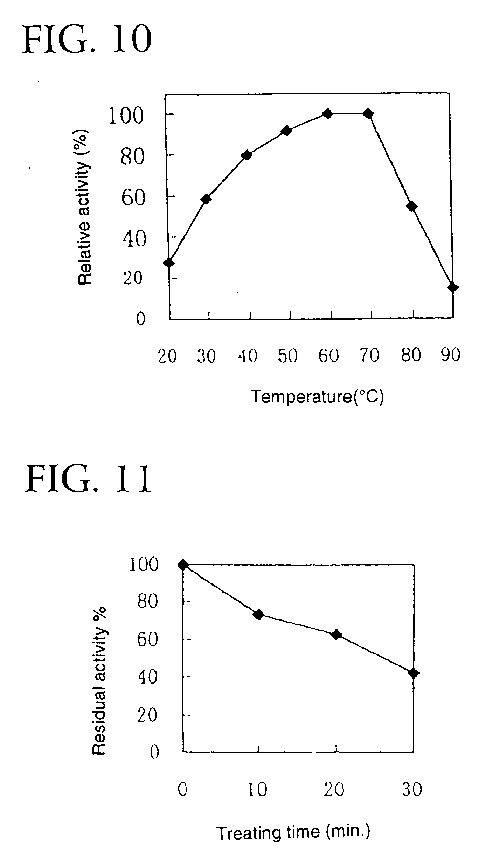

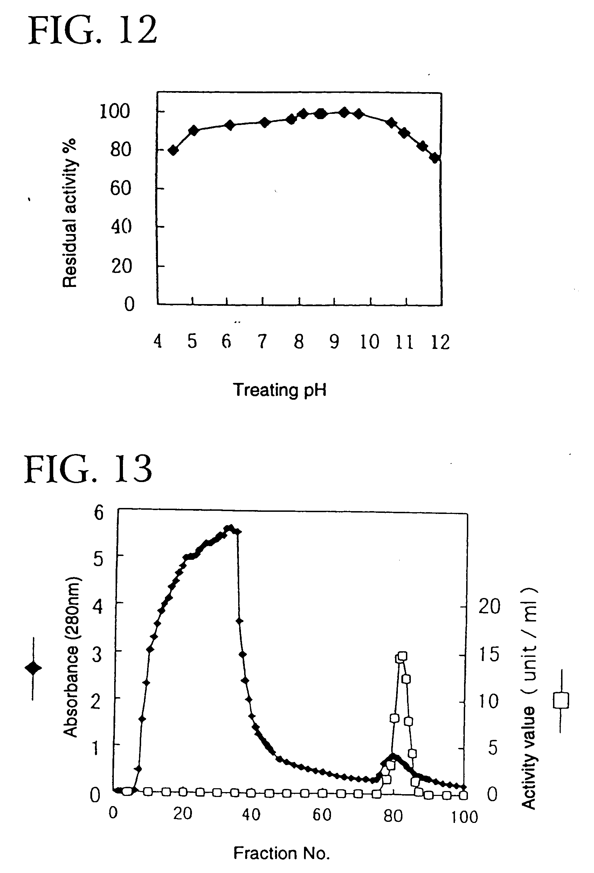

The enzyme can progress polyphenol oxidation reaction in a wide pH range of 6 to 12, and the reaction pH is preferably 8 to 11, and more preferably 8.5 to 10, with the optimum pH around pH 9, to characteristically catalyze the oxidation reaction in an alkaline region (Fig.9). For reaction at pH 8.7 for 10 minutes, additionally, the optimum temperature is around 70°C (Fig.10). Further, almost 50 % of the activity remained after 30-min heating processes for various given periods, for example 30 minutes (Fig.11). Still furthermore, the residual activity after 30-min processes at 30°C in buffer solutions of various pHs were stable in a wide pH range. These results verify the oxidation reaction of the enzyme derived from *Myrothecium roridum* SD3002 in various solutions at medium to low temperature in a wide pH range from neutrality to alkalinity.

The polyphenol oxidase of the present invention can be used in combination with conventional such enzymes with the optimum pHs in an acidic region. In other words, the use of the polyphenol oxidase of the present invention in combination with conventionally known polyphenol oxidases with the optimum reaction pHs in an acidic region can effect polyphenol oxidase reaction in a wide pH range from acidity to alkalinity. When the enzymes are used in combination for such purpose, the activity level of a polyphenol oxidase with the optimum reaction pH in an acidic region and the activity level of the polyphenol oxidase of the present invention are at a mixing ratio of preferably 1 : 10 to 10 : 1, more preferably 1 : 3 to 3 : 1. The polyphenol oxidase of the present invention is useful, additionally because a polyphenol oxidase reaction can be attained in a wide pH range in such manner.

[Enzyme activity assay]

In accordance with the present invention, the polyphenol oxidation activity of the polyphenol oxidase was assayed during reaction in an aqueous solution containing 20 ppm syringaldazine and 100 mM Tris-HCl buffer solution, pH 8.7 at 20°C, by measuring the absorbance at 525 nm. Then, the activity level oxidizing 1 μmol of syringaldazine per minute was defined as 1 unit (abbreviated as ì1 Uî hereinafter).

[Utilities]

The use of the novel polyphenol oxidase with the optimum reaction region at an alkaline pH includes for example the application to bleaching.

The use of polyphenol oxidases for bleaching are described for example WO 91-05839, DE 4008894, Japanese Patent Laid-open No. 64-60693 and the like. However, cleaning procedures such as laundry washing are generally carried out at an alkaline pH, and when oxidization bleaching in the presence of hydrogen peroxide is simultaneously carried out, the cleaning procedures are preferably carried out at an alkaline pH so as to promote the bleaching action of hydrogen peroxide as well. Conventional polyphenol oxidases with the optimum reaction pHs at an acidic pH have hardly been applied to these utilities, substantially. The polyphenol oxidase of the present invention is useful for developing the applicability of polyphenol oxidase to the field of alkaline cleaning and alkaline bleaching.

Oxidation bleaching by hydrogen peroxide is currently used widely in cleaning and laundry washing. However, hydrogen peroxide does not have sufficient bleaching strength at low temperature below 60°C. So as to improve the strength, a peracid precursor is used in combination with hydrogen peroxide, but the bleaching strength thereof at low temperature below 40°C is not satisfactory. Hence, a bleaching system with higher effect has been needed. Therefore, a variety of methods for enzymatically promoting bleaching have been proposed conventionally. The presence of the polyphenol oxidase disclosed herein in combination with one or a plurality of substances with peroxidase action, such as peroxidase, lignin peroxidase, and manganese peroxidase, can facilitate the oxidation bleaching at an alkaline pH, which apparently indicates the usefulness of the present invention.

Hydrogen peroxide widely used for oxidation bleaching is a costly oxidant, and further, hydrogen peroxide precursors, peracid precursors and peracids frequently used as cleaning agents are more costly oxidants. Additionally, the use of oxidase and a substrate thereof can generate hydrogen peroxide enzymatically, but such hydrogen peroxide generation system is also believed as a costly oxidant. The polyphenol oxidase of the present invention can promote oxidation bleaching, if used concurrently with air, oxygen, ozone, hydrogen peroxide, a hydrogen peroxide precursor, a peracid precursor or a peracid, singly or in combination of a plurality thereof. Thus, the usefulness of the polyphenol oxidase of the present invention capable of achieving oxidation bleaching involving the efficacious use of oxidants is apparently shown.

Amongst oxidants, the hydrogen peroxide precursor is dissolved in water to generate perhydroxyl ion. Such substance includes perborate (perboric acid salt) monohydrate or tetrahydrate, percarbonate (percarbonate salt), perborax, sodium perpyrophosphate, perbenzoic acid, urea-hydrogen peroxide reaction product, melamine-$H_2O_2$ product, citrate perhydrate and the like; preference is given to perborate and percarbonate, in particular. As the hydrogen peroxide precursor, still further, a hydrogen peroxide generation system of oxidase and a substrate thereof can be used. Examples of such oxidase include glucose oxidase, alcohol oxidase, glycerol oxidase, amine oxidase, amino acid oxidase, D-amino acid oxidase, aryl alcohol oxidase, aldehyde oxidase, galactose oxidase, sorbose oxidase, urate oxidase, xanthine oxidase, and cholesterol oxidase; preference is given to glucose oxidase and alcohol oxidase, in particular.

Additionally, the peracid precursor includes organic compounds with a reactive acyl group, or carboxylate ester, carboxylic anhydride or acetate salt, such as TAED (tetraacetylethylenediamine), TAMD (tetraacetylmethylenediamine), TAGU (tetraacetylglycoluril), DADHT (diacetyldioxohexahydrotriazine), SNOBS (sodium nonanoyloxybenzene sulfonate), ISONOBS (sodium isononanoyloxybenzene sulfonate), succinic anhydride, benzoic anhydride, phthalic anhydride, PAG (glucose pentaacetate), and xylose tetraacetate; preference is given to TAED and SNOBS, in particular.

Further, the peracid includes for example DPDDA (diperoxydodecanedioic acid), DPIPA (diperoxyisophthalic acid), MMPPH (magnesium monoperoxyphthalate hexahydrate), and NAPAA (nonylamidoperoxyadipic acid).

The polyphenol oxidase of the present invention can be used in combination with a variety of cleaning agents, detergents or surfactants, whereby a cleaning composition or a detergent composition blended with the polyphenol oxidase of the present invention is provided. Representative examples of such cleaning- or detergent composition include a cleaning- or detergent composition comprising at least one or more selected from the group consisting of a surfactant at 10 to 50 % by weight per the weight of the cleaning- or detergent composition, a builder at 0 to 50 % by weight per the weight, an alkaline agent or an inorganic electrolyte at 1 to 50 % by weight per the weight, a re-staining preventive agent at 0.1 to 10 % by weight per the weight, an enzyme, a bleaching agent, a fluorescent dye, a caking preventive agent and an antioxidant.

As the surfactant, any surfactant routinely blended as a detergent composition can be used, including soap, for example, a linear or branched alkyl- or alkenyl sulfate; an amide sulfate; an aliphatic sulfated product such as an alkyl- or alkenyl ether sulfate, having a linear or branched alkyl or alkenyl group and with addition of a single component or a plurality of components from ethylene oxide, propylene oxide and butylene oxide; an alkyl sulfonate; an amide sulfonate; a dialkyl sulfosuccinate; an aliphatic sulfonate such as sulfonates of alpha-olefin, vinylidene-type olefin and inner olefin; an aromatic sulfonate such as a linear or branched alkylbenzene sulfonate; an alkyl- or alkenyl ether carbonate or amide, having a linear or branched alkyl group or alkenyl group and with addition of a single component or a plurality of components from ethylene oxide, propylene oxide and butylene oxide; an alpha-sulfofatty acid salt or ester; an amino acid-type surfactant; a phosphate ester-type surfactant such as an alkyl- or alkenyl acid phosphate ester or an alkyl- or alkenyl phosphate salt; a sulfonate-type amphoteric surfactant; a betaine-type amphoteric surfactant; an alkyl- or alkenyl ether or alcohol, having an alkyl- or alkenyl group and with addition of a single component or a plurality of components from ethylene oxide, propylene oxide and butylene oxide; a polyoxyethylene alkylphenyl ether having a linear or branched alkyl- or alkenyl group and with addition of a single component or a plurality of components from ethylene oxide, propylene oxide and butylene oxide; a higher fatty acid alkanol amide or an alkylene oxide adduct thereof; a sucrose fatty acid ester; a fatty acid glycerin monoester; an alkyl- or alkenyl amine oxide; and a tetraalkylammonium salt-type cationic surfactant. As the ion pair for nonionic surfactants, sodium ion or potassium ion is preferable. These surfactants are used singly or in a mixture of two or more thereof.

As the builder and the alkaline agent or the inorganic electrolyte, use is made of phosphates such as orthophosphate, pyrophosphate, tripolyacid salt, metaphosphate, hexametaphosphate and phytic acid salt; phosphonates such as ethane-1,1-diphosphonate and derivatives thereof, ethanehydroxy-1,1,2-triphosphonate, ethane-1,2-dicarboxy-1,2-diphosphonate, and methanehydroxyphosphonate; phosphonocarboxylates such as 2-phosphonobutane-1,2-dicarboxylic acid, 1-phosphonobutane-2,3,4-tricarboxylic acid, alpha-methylphosphonosuccinic acid; amino acid salts such as aspartic acid and glutamic acid; aminopolyacetates such as nitrilotriacetate, ethylenediaminetetraacetate and diethylenetriaminepentaacetate; polymer electrolytes such as polyacrylic acid, polyitaconic acid, polymaleic acid, maleic anhydride copolymer, and carboxymethyl cellulose salt; non-dissociative polymers such as polyethylene glycol and polyvinyl alcohol; carboxymethylated products such as diglycolic acid, oxydisuccinic acid, carboxymethyloxysuccinic acid, gluconic acid, citric acid, lactic acid, tartaric acid, sucrose, and lactose; carboxymethylated product of pentaerythritol; salts of organic acids, such as benzene polycarboxylate, oxalic acid, malic acid, oxydisuccinic acid, and gluconic acid; aluminosilicates such as zeolite; inorganic salts such as carbonate, sesquicarbonate, sulfate and metasilicate to be used in the form of alkali metal salt; additionally, use is made of organic substances such as starch and urea and inorganic compounds such as sodium chloride and bentonite; still further, use is made of triethanol amine, diethanol amine, monoethanol amine and triisopropanol amine, as an organic alkaline agent.

As has been described above, the detergent composition of the present invention contains a surfactant and the polyphenol oxidase and the like as the structural components thereof, but if necessary, the detergent composition can contain an amphoteric surfactant, a bleaching agent for example perborate and percarbonate, a dye, a builder, a re-staining preventive agent such as polyethylene glycol, polyvinyl alcohol, polyvinyl pyrrolidone and carboxymethyl cellulose, a caking preventive agent, an antioxidant, and other enzymes for example other oxidases, peroxidase, protease, lipase, amylase and cellulase.

Any method may be adopted for blending enzymes such as the polyphenol oxidase of the present invention in a detergent composition, but the blending procedure of enzymes in ultrafine powder is not preferable for labor hygiene of detergent users and workers engaged in the detergent industry, because of dust generation during handling of detergents. Therefore, preferably, the enzymes are preliminarily formulated in a solution state or a form capable of suppressing dust generation.

The formulation procedure is carried out according to common Marmeís granulation, extrusion granulation, fluid granulation and centrifugation fluid granulation and other methods, and the form of enzymes blended in the detergent composition in accordance with the present invention is not specifically limited to the forms formulated by these methods.

For the purpose of delignination and bleaching, biopulping and biobleaching are a field of useful application, comprising inoculating the bacterial strain of the present invention at a part of the pulping process to generate the polyphenol oxidase of the present invention or directly adding the enzyme specimen of the present invention to act on chip or crudely ground pulp. In such case, the polyphenol oxidase with the optimum reaction pH in an alkaline region in accordance with the present invention can reduce the amount of chemical solutions to be used for pH adjustment, compared with conventional polyphenol oxidases with the optimum reaction pHs in an acidic region, whereby cost reduction can be attained.

As natural products containing polyphenol in the backbone structure, plant dyes such as flavonoids, xanthones and melanines and lignin have been known, and polyphenol oxidases have an oxidizing action for these natural products. Further, polyphenol oxidases can utilize, as a reaction substrate, AOX with toxicity under serious concern, such as dichlorophenol and trichlorophenol. Hence, the polyphenol oxidase of the present invention is useful for the disposal of liquid waste containing these natural products and non-natural products.

A biosensor using the polyphenol oxidase of the present invention is useful because the biosensor reflecting the characteristics of the enzyme can be used for monitoring a variety of aqueous solutions with pHs in neutral to alkaline regions and aromatic compounds in organic solvents.

By utilizing the reactivity of phenoxy radical generated by the polyphenol oxidase of the present invention, sterilization and inactivation of microorganisms and viruses in neutral to alkaline pH regions can be carried out efficiently. More specifically, strong sterilizing activity can be imparted from the phenoxy radical enzymatically generated in addition to the sterilizing potency of the polyphenol oxidase substrate of itself. Additionally, when the resulting sterilized matter is thereafter put in contact to human bodies or incorporated by human bodies or is discharged into environment, the polyphenol oxidase substrate is modified into a substance with reduced toxicity through oxidation, and therefore, both the sterilizing potency at a needed point and the safety thereafter can be attained, which indicates higher usefulness.

For polymer synthesis using phenoxy radicals or quinones generated by polyphenol oxidase, reactions applicable thereto can potentially be enlarged, which indicates the usefulness of the polyphenol oxidase of the present invention.

For oxidizing a substance group containing readily oxidizable polyphenol such as catechol, the use of the polyphenol oxidase at an alkaline pH, capable of autonomous oxidation reaction and enzymatically catalyzed oxidation reaction of the polyphenol at an alkaline pH is extremely useful for greatly efficient deoxygenation.

BEST MODE FOR CARRYING OUT THE INVENTION

The present invention is now more specifically described with reference to the following representative examples by way of simple illustration. The present invention is not limited only to these examples.

Example 1: Culturing and concentration of *Myrothecium_verrucaria* strain SD3001

Two-liter flask was used as a culturing apparatus. *Myrothecium verrucaria* strain SD3001 (Accession No. FERM P-14955) was inoculated in 400 ml of a culture medium containing 0.5 % glucose, 0.1 % $(NH_4)_2SO_4$, 1.34 % $Na_2HPO_4$ · $12H_2O$, 0.3 % $KH_2PO_4$, 0.1 % NaCl, 0.1 % peptone, 0.01 % yeast extract, and 0.1 mM $CuSO_4$, to which medium was preliminarily added 2N NaOH for adjustment to pH 8, for culturing under stirring at 27°C for 3 days. After culturing, the culture broth sterilized through centrifugation at 4°C was recovered. Then, the resulting culture broth was concentrated and purified through an ultrafiltration membrane to recover an aqueous concentrated solution (20 U/ml) crudely purified as a fraction range of a molecular weight of 10,000 to 100,000.

Example 2: Crude purification

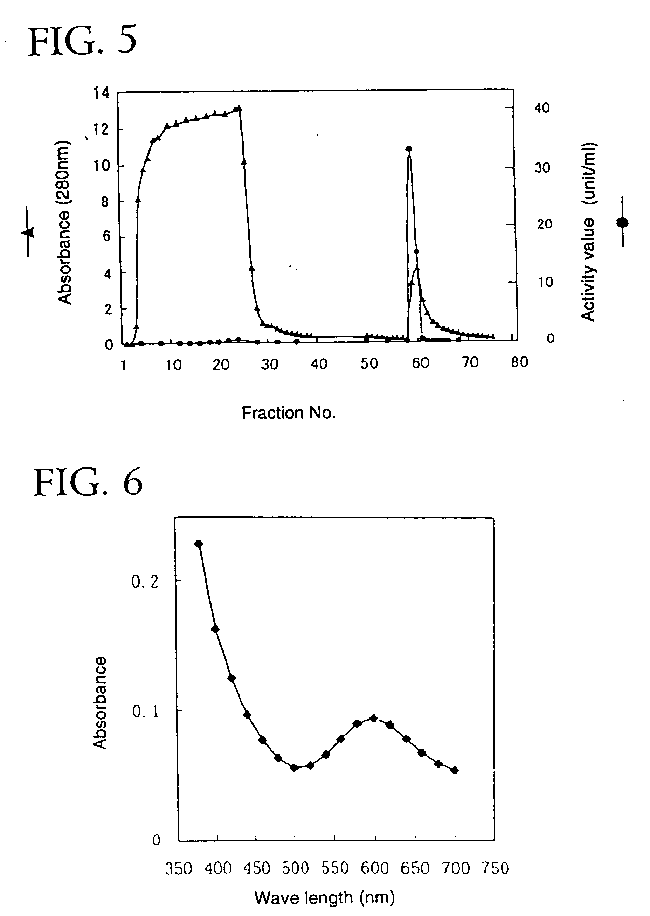

Into the void space in the upper part of a DEAE-Cellulofine A -800 m (manufactured by Biochemical Industry, K.K.) column (⌀20 mm, 50 cc) equilibrated with 1.34 % $Na_2HPO_4$ · $12H_2O$, 0.3 % $KH_2PO_4$, and 0.1 % NaCl was applied the culture broth in sterilization described in Example 1, and on the initiation of elution, the recovery of the eluate was started as each fraction of 15 ml. After applying 345 ml of the culture broth, column washing with a buffer solution of the same composition as used for equilibration and elution with a stepwise gradient of the NaCl concentration up to 0.15 M were conducted. Fig.5 depicts a pattern of the absorbance at 280 nm and polyphenol oxidase activity of each fraction. Among the fractions, Nos.1 to 55 correspond to 0.1 % NaCl-eluted fractions; Nos. 56 to 75 correspond to 0.15 M NaCl-eluted fractions. Intense oxidase activity was detected in Nos.59 and 60. The details of the Fractions Nos. 59 and 60 are shown in

Table 1

| Fraction No. | Absorbance at 280 nm | Activity (unit/ml) | Purification ratio |
|---|---|---|---|
| 59 | 3.32 | 32.4 | 112 |
| 60 | 4.17 | 15.1 | 42 |

The absorbance of Fraction No.59 was measured in a range of 380 nm to 700 nm. Consequently, the absorption peak was observed around 600 nm (Fig.6).

Example 3: Substrate specificity

Using the crudely purified polyphenol oxidase as Fraction No.59 described in Example 2, the substrate specificity of the oxidation of polyphenol compounds was examined, by measuring the difference in oxygen consumption between aqueous solutions containing a 0.05 mM substrate and 100 mM Tris-HCl buffer solution, pH 8.7 with addition and without addition of the enzyme at room temperature (20°C). The results are shown in

Table 2

| Substrate | Oxidation reaction |
|---|---|
| 4-Anisidine | +++ |
| o-Phenylenediamine | +++ |
| Syringaldazine | +++ |
| Syringic acid | +++ |
| Ferulic acid | ++ |

Example 4: Molecular weight

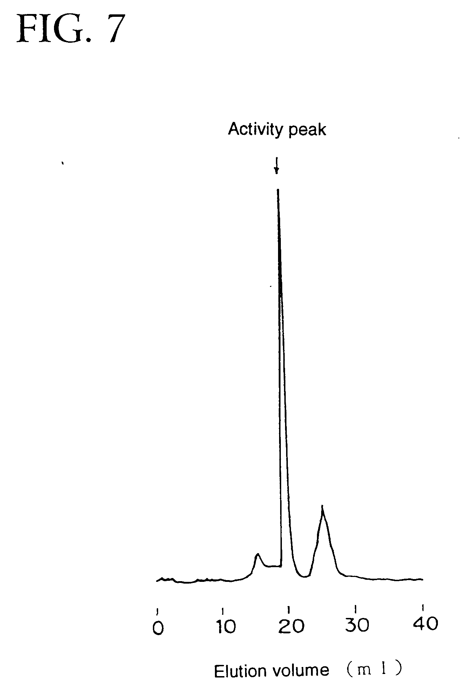

Molecular weight was measured by GFC (gel filtration chromatography).
By HPLC using a GFC column (Shodex PROTEIN KW-802.5, two series) equilibrated with 1.34 % $Na_2HPO_4$ · $12H_2O$, 0.3 % $KH_2PO_4$, and 1 % NaCl at a flow rate of 1.0 ml/min along with a UV detector (280 nm), the analysis, isolation and activity assay of the crudely purified polyphenol oxidase as Fraction No.59 described in Example 2, was carried out. The activity peak of the polyphenol oxidase corresponded to a molecular weight of 62,000. As a molecular weight marker protein, MW-Marker (HPLC; manufactured by Oriental Industry, K.K. ) was used. The elution pattern of Fraction No.59 by the GFC analysis (HPLC) is shown in Fig.7.

Example 5: Bleaching treatment of cloth with tea stain

In 10 ml of 50 mM sodium carbonate buffer solution, pH 9.0 containing 0.1 % Tween 80 and 0.02 % active zeolite powder was immersed tea-stained cloth of a size of 5 cm x 5 cm, followed by addition of a blend product generated by preliminarily blending the crudely purified polyphenol oxidase as Fraction No.59 described in Example 2 with other bleaching agents, and the resulting mixture was stirred at 30°C for 40 minutes for cleaning and bleaching treatment. Thereafter, the cloth was washed in water and dried in air, to measure then the values of Y, y and x on the XYZ color specification system [CIE (Commission Internationale de l'Eclairage) Standard color specification] by a color differential colorimeter (CR-200; manufactured by MINOLTA); additionally, the value Z was calculated by the formula $[Z = (1-x- y)Y/y]$ . Then, the improvement of whitening degree was evaluated on the basis of the difference in Z ($\Delta$ Z) between prior to and after the process. The results are shown in Table 3.

The cloth with tea stain was prepared, by placing 160 g of a commercially available type of tea leave(Hotel Restaurant Blend Ceylon; Nippon Tea, K.K. ) in 5 liters of boiling water, boiling the water for 5 minutes, removing the tea leaves through filtration, boiling the filtrate for 5 minutes, and thereafter placing cotton white cloth therein prior to another 5-min boiling, drawing out and drying the cloth, washing the cloth sufficiently in water and drying again the cloth.

Table 3

| Additives | $\Delta$Z value |
|---|---|
| No additive | 0.2 |
| 1 U/ml crudely purified enzyme solution | 1.2 |
| 200 ppm hydrogen peroxide | 4.8 |
| 1 U/ml crudely purified enzyme solution + 200 ppm hydrogen peroxide | 6.5 |
| 200 ppm percarbonate · Na salt | 2.8 |
| 1 U/ml crudely purified enzyme solution + 200 ppm percarbonate · Na salt | 4.6 |
| 200 ppm percarbonate · Na salt + 50 ppm TAED | 6.1 |
| 1 U/ml crudely purified enzyme solution + 200 ppm percarbonate · Na salt + 50 ppm TAED | 8.1 |
| 200 ppm percarbonate · Na salt + 50 ppm SNOBS | 7.2 |
| 1 U/ml crudely purified enzyme solution + 200 ppm percarbonate · Na salt + 50 ppm SNOBS | 8.7 |

Example 6: Culturing, concentration and crude purification in 5-liter culture tank

In a 5-liter tank containing 3 liters of a culture medium containing 0.5 % glucose, 0.1 % $NaNO_3$, 1.34 % $Na_2HPO_4$ · $12H_2O$, 0.3 % $KH_2PO_4$, 0.1 % NaCl, 0.2 % peptone, 200 ppm yeast extract, 0.01 % $MgSO_4$ · $7H_2O$ and 0.1 mM $CuSO_4$ , after adjustment to pH 8 by adding 10 % NaOH to the medium, was inoculated *Myrothecium verrucaria* SD3001 (Accession No. FERM P-14955), for culturing under stirring at 28°C for 3 days. After culturing, the culture broth sterilized through centrifugation at 4°C was recovered at a yield of 2.5 liters.

Then, a part of the resulting culture broth was concentrated and purified as a fraction of a molecular weight of 10,000 or more, by Minitane Ultrafiltration System (Manufactured by Millipore) using Minitane Filter Bucket. The fraction was further subjected to DEAE-Cellulofine A-800m column chromatography, and the eluted active fractions were again concentrated as a fraction of a molecular weight of 10,000 or more by the Minitane Ultrafiltration System. Still further, the re-concentrated solution was dialyzed against 200 ppm $NH_4HCO_3$ and was then subjected to freeze-drying to recover a crudely purified product as a freeze-dried matter. The polyphenol oxidase activity of the freeze-dried product was 10 U/mg.

Example 7: Treatment of cloth with stain by enzyme-containing detergent

To 10 g of a model detergent comprising 25 % by weight of linear sodium alkylbenzene sulfonate (LAS), 5 % by weight of polyoxyethylene lauryl ether, 15 % by weight of sodium tripolyphosphate, 6 % by weight of sodium silicate, 1 % by weight of sodium carboxymethyl cellulose and 48% by weight of $Na_2SO_4$ was added 0.1 g of the freeze-dried product described in Example 6, and the resulting product was defined as enzyme-blended detergent. The model detergent with no addition of the freeze-dried, product was defined as non-enzyme-blended detergent.

Additionally, cloth with stain was prepared by attaching 0.2 ml of 100 ppm Evans Blue (Manufactured by Wako Pure Chemicals, K.K.) onto the center of white cotton cloth (5 cm x 5 cm).

After placing a piece of the cloth with the stain and 10 ml of water in a 500-ml beaker and further adding therein 10 mg of the enzyme-blended detergent or the non-enzyme-blended detergent, cleaning process was effected under 12-min stirring. The cloth with the stain after the process was washed in water and dried in air, to calculate then the Z value by a color differential colorimeter (CR-200; manufactured by MINOLTA) in the same manner as in Example 5; and the improvement of whitening degree by the enzyme-blended detergent was larger by 6 points than the improvement of whitening degree by the non-enzyme-blended detergent.

Example 8: Bleaching treatment of crudely purified pulp

A beaker of 500-ml volume was used as a culturing apparatus. *Myrothecium verrucaria* strain SD3001 (Accession No. FERM P-14955) was inoculated in 50 ml of a culture medium containing 0.3 % glucose, 0.05 % $(NH_4)_2SO_4$, 1.34 % $Na_2HPO_4 \cdot 12H_2O$, 0.3 % $KH_2PO_4$, 0.1 % NaCl, 0.02 % peptone, 50 ppm yeast extract, 0.02 mM $CuSO_4$, and 8 % broad-leaves unbleached craft pulp (LUKP) (Kappa value of 18), which medium was preliminarily adjusted to pH 8.5 by the addition of 2N NaOH, for stationary culture under stirring at 27°C for 4 days. Thereafter, the Kappa value was measured. It was verified that the value was improved by 5 points.

Example 9: Mixing of the enzyme derived from *Myrothecium verrucaria* SD3001 with conventional polyphenol oxidase

Using a mixture of equal volumes of a 0.1 U/ml solution of a polyphenol oxidase (derived from *Rigidoporus zonalis*; available from TaKaRa, K.K.) commercially available as a reagent and a 0.2 U/ml solution of the polyphenol oxidase of the present invention, the activity was measured at a variety of pHs. As shown in Fig.8, the oxidation reaction rapidly progressed in a wide pH range of pH 5 to 11.

Example 10 Culturing and concentration of *Myrothecium roridum* strain SD3002

Two-liter flask was used as a culturing apparatus. *Myrothecium roridum* strain SD3002 (Accession No. FERM P-15255) was inoculated in 550 ml of a culture medium containing 1 % glucose, 0.2 % $NH_4Cl$, 1.34 % $Na_2HPO_4 \cdot 12H_2O$, 0.3 % $KH_2PO_4$, 0.1 % NaCl, 0.2 % peptone, 0.05 % $MgSO_4 \cdot 7H_2O$ and 0.02 mM $CuSO_4$, which medium was preliminarily adjusted to pH 8 by the addition of 2N NaOH, for culturing under stirring at 27°C for 90 hours. After culturing, the culture broth sterilized through centrifugation at 4°C was recovered. Then, the resulting culture broth was concentrated and purified through an ultrafiltration membrane, to recover an aqueous concentrated solution (0.4 U/ml) crudely purified as a fraction range of a molecular weight of 10,000 to 100,000.

Example 11: Crude purification

Into the void space in the upper part of a DEAE-Cellulofine A -800 m (manufactured by Biochemical Industry, K.K.) column ($\varnothing$26 mm, 90 cc) equilibrated with 1.34 % $Na_2HPO_4 \cdot 12H_2O$, 0.3 % $KH_2PO_4$, and 0.1 % NaCl was applied the concentrated culture broth in sterilization, described in Example 10, and on the initiation of elution, the recovery of the eluate was started as each fraction of 16 ml. After applying 480 ml of the culture broth, column washing with a buffer solution of the same composition as used for equilibration and elution with a step-wise gradient of the NaCl concentration up to 0.12 M were conducted. Fig.13 depicts a pattern of the absorbance at 280 nm and polyphenol oxidase activity of each fraction. Among the fractions, Nos.1 to 70 correspond to 0.1 % NaCl-eluted fractions; Nos. 71 to 100 correspond to 0.12 M-NaCl eluted fractions. Intense oxidase activity was detected in Nos.80 to 84.

Example 12 Substrate specificity

Using the crudely purified polyphenol oxidase as Fraction No.82, described in Example 11, the substrate specificity of the oxidation of polyphenol compounds was examined, by measuring the difference in oxygen consumption between aqueous solutions containing a 0.05 mM substrate and 100 mM Tris-HCl buffer solution, pH 8.7 with addition and without addition of the enzyme at room temperature (20°C). The results are shown in Table 4.

Table 4

| Substrate | Oxidation reaction |
|---|---|
| Syringaldazine | ++ |
| 4-Anisidine | + |
| o-Phenylenediamine | + |
| Ferulic acid | + |

Example 13 Molecular weight

Molecular weight was measured by GFC (gel filtration chromatography). More specifically, by HPLC using a GFC column (Shodex PROTEIN KW-802.5, two series) equilibrated with 1.34 % $Na_2HPO_4 o 12H_2O$, 0.3 % $KH_2PO_4$, and 1 % NaCl at a flow rate of 1.0 ml/min along with a UV detector (280 nm), the analysis, isolation and activity assay of the crudely purified polyphenol oxidase as Fraction No.82, described in Example 11, was carried out. The activity peak of the polyphenol oxidase corresponded to a molecular weight of 60,000. As a molecular weight marker protein, MW-Marker (HPLC; manufactured by Oriental Industry, K.K. ) was used.

Example 14 Culturing and crude purification in 5-liter culture tank

Into two 5-liter flasks individually containing 1 liter of a culture medium containing 1 % glucose, 0.2 % $NH_4Cl$, 1.34 % $Na_2HPO_4$ • $12H_2O$, 0.3 % $KH_2PO_4$, 0.1 % NaCl, 0.2 % peptone, 0.05 % $MgSO_4$ • $7H_2O$ and 0.02 mM $CuSO_4$ , which medium was adjusted to pH 8 by the addition of 2N NaOH, was inoculated *Myrothecium roridum* SD3002 (Accession No. FERM P-15255), for culturing under stirring at 28°C for 3 days. After culturing, the culture broth sterilized through centrifugation at 4°C was recovered at a yield of 1.6 liters.

Then, a part of the resulting culture broth was concentrated and purified as a fraction of a molecular weight of 10,000 or more, by Minitane Ultrafiltration System (Manufactured by Millipore) using Minitane Filter Bucket. The fraction was further subjected to DEAE-Cellulofine A-800m column chromatography, and the eluted active fractions were again concentrated as a fraction of a molecular weight of 10,000 or more by the Minitane Ultrafiltration System. Still further, the re-concentrated solution was dialyzed against 200 ppm $NH_4HCO_3$ and was then subjected to freeze-drying to recover a crudely purified product as a freeze-dried matter. The polyphenol oxidase activity of the freeze-dried product was 5 U/mg.

Example 15 Treatment of cloth with stain by enzyme-containing detergent

To 1 g of a model detergent comprising 25 % by weight of linear sodium alkylbenzene sulfonate (LAS), 5 % by weight of polyoxyethylene lauryl ether, 15 % by weight of sodium tripolyphosphate, 6 % by weight of sodium silicate, 1 % by weight of sodium carboxymethyl cellulose and 48% by weight of $Na_2SO_4$ was added 0.01 g of the freeze-dried product described in Example 14, and the resulting product was defined as enzyme-blended detergent. The model detergent with no addition of the freeze-dried product was defined as non-enzyme-blended detergent.

Additionally, cloth with stain was prepared, by attaching 0.2 ml of 100 ppm Evans Blue (Manufactured by Wako Pure Chemicals, K.K.) onto the center of white cotton cloth (5 cm x 5 cm).

After placing a piece of the cloth with the stain and 10 ml of water in a 500-ml beaker and further adding therein 10 mg of the enzyme-blended detergent or the non-enzyme-blended detergent, cleaning process was effected under 12-min stirring. The cloth with the stain after the process was washed in water and dried in air, to measure then the values of Y, y and x representing the same as described above, by a color differential colorimeter (CR-200; manufactured by MINOLTA); additionally, the difference in value Z between prior to and after the process was calculated on the basis of the formula [Z = (1-x-y)Y/y] . The improvement of whitening degree by the enzyme-blended detergent was larger by 3 points than the improvement of whitening degree by the non-enzyme-blended detergent.

Example 16 Mixing of the enzyme derived from *Myrothecium roridum* SD3002 with conventional polyphenol oxidase

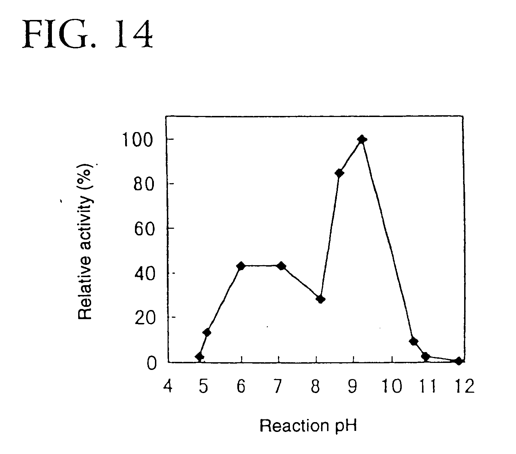

Using a mixture of equal volumes of a 0.1 U/ml solution of a polyphenol oxidase (derived from *Rigidoporus zonalis*; available from TaKaRa, K.K.) commercially available as a reagent and a 0.2 U/ml solution of the polyphenol oxidase of the present invention, the activity was measured at a variety of pHs. As shown in Fig.14, the oxidation reaction rapidly progressed in a wide pH range of pH 5 to 11.

Example 17 Measurement of isoelectric point

Using the freeze-dried product described in Example 14, isoelectric electrophoresis using Rhotophore System (manufactured by BIO-RAD Company) was carried out for measuring the isoelectric point. As the buffer solution, Pharmalite (pH 2.5 - 5) (available from Sigma, Co.) was used.

Consequently, the isoelectric point of the polyphenol oxidase derived from *Myrothecium roridum* SD3002 (Accession No. FERM P-15255) was 4.1 ± 0.5. Using the freeze-dried product described in Example 6, the same method was practiced to measure the isoelectric point of the polyphenol oxidase derived from *Myrothecium verrucaria* SD3001 (Accession No. FERM P-14955), which was 4.5 ± 0.5.

INDUSTRIAL APPLICABILTY

In accordance with the present invention, as has been described above, the polyphenol oxidase with the optimum reaction pH in an alkaline region above pH 8 is provided; and by using the oxidase, enzymatic oxidation in an alkaline pH region can be attained, whereby the applicable range of polyphenol oxidase is enlarged, including the oxidative treatment of polyphenol substances and coloring agents, cleaning and bleaching.

Additionally, the method for producing a polyphenol oxidase in accordance with the present invention can efficiently produce the polyphenol oxidase of the present invention.

The *Myrothecium verrucaria* SD3001 and *Myrothecium roridum* SD3002 are effective for producing the polyphenol oxidase of the present invention, and furthermore, the direct use thereof can produce great effect on pulp bleaching process.

By using the polyphenol oxidase of the present invention, effective processes including treatment of coloring substances, treatment of paper, pulp or fiber, bleaching process, cleaning process or sterilization and inactivation process of microorganisms and viruses are provided.

**Claims**

1. A polyphenol oxidase with the optimum reaction pH in an alkaline region above pH 8, which is derived from a microorganism.

2. A polyphenol oxidase according to claim 1, which is derived from the genus *Myrothecium*.

3. A polyphenol oxidase according to claim 1 or 2, having the following properties:

   (1) action;
   oxidizing polyphenol;
   (2) optimum reaction pH;
   having the optimum reaction pH around pH 8.5 to 9;
   (3) optimum reaction temperature;
   having the optimum reaction temperature at about 60°C;
   (4) molecular weight;
   the molecular weight measured by gel filtration chromatography analysis is about 62,000; and
   (5) isoelectric point measured by isoelectric electrophoresis is 4.5 ± 0.5.

4. A polyphenol oxidase according to claim 1, 2 or 3, which can be recovered from *Myrothecium verrucaria*.

5. A polyphenol oxidase according to claim 1, 2 or 3, which can be recovered from *Myrothecium verrucaria* strain SD3001 (Accession No. FERM P-14955).

6. A polyphenol oxidase according to claim 1 or 2, having the following properties:

   (1) action;
   oxidizing polyphenol;
   (2) optimum reaction pH;
   having the optimum reaction pH around pH 9;
   (3) optimum reaction temperature;

having the optimum reaction temperature around 70°C;
(4) molecular weight;
the molecular weight measured by gel filtration chromatography analysis is about 60,000; and
(5) isoelectric point measured by isoelectric electrophoresis is 4.1 ± 0.5.

7. A polyphenol oxidase according to claim 1, 2 or 6, which can be recovered from *Myrothecium roridum*.

8. A polyphenol oxidase according to claim 1,2 or 6, which can be recovered from M*yrothecium roridum* strain SD3002 (Accession No. FERM P-15255).

9. A method for treating phenol compounds, alkoxyl group-containing aromatic compounds, halogenated phenol compounds or aromatic amine compounds, comprising making a polyphenol oxidase according to any one of claims 1 to 8 act thereon.

10. A method for treating coloring substances, comprising making a polyphenol oxidase according to any one of claims 1 to 8 act thereon.

11. A method for treating microorganisms or viruses, comprising making a polyphenol oxidase according to any one of claims 1 to 8 act thereon.

12. A method for treating paper, pulp or fiber, comprising making a polyphenol oxidase according to any one of claims 1 to 8 act thereon.

13. A method for utilizing a polyphenol oxidase according to any one of claims 1 to 8, characteristically using the polyphenol oxidase in combination with a cleaning agent, a detergent or a surfactant.

14. A method for utilizing a polyphenol oxidase according to any one of claims 1 to 8, characteristically using the polyphenol oxidase in combination with a substance with peroxidase action.

15. A method for utilizing a polyphenol oxidase according to any one of claims 1 to 8, characteristically using the polyphenol oxidase in combination with air, oxygen, ozone, hydrogen peroxide, a hydrogen peroxide precursor, a peracid precursor or peracid singly or in combination of a plurality thereof.

16. A method for utilizing a polyphenol oxidase according to any one of claims 1 to 8, characteristically using the polyphenol oxidase in combination with a substrate thereof.

17. A method for producing a polyphenol oxidase according to claim 1 to 8, comprising culturing the bacteria of the genus *Myrothecium*.

18. A method for producing a polyphenol oxidase according to claim 17, wherein the bacteria of the genus *Myrothecium* belong to *Myrothecium verrucaria*.

19. A method for producing a polyphenol oxidase according to claim 17, wherein the bacteria of the genus *Myrothecium* belong to *Myrothecium verrucaria* strain SD3001 (Accession No. FERM P-14955) or a variant thereof.

20. A method for producing a polyphenol oxidase according to claim 17, wherein the bacteria of the genus *Myrothecium* belong to *Myrothecium roridum*.

21. A method for producing a polyphenol oxidase according to claim 17, wherein the bacteria of the genus *Myrothecium* belong to *Myrothecium roridum* strain SD3002 (Accession No. FERM P-15255) or a variant thereof.

22. *Myrothecium verrucaria* strain SD3001 (Accession No. FERM P-14955).

23. *Myrothecium roridum* strain SD3002 (Accession No. FERM P-15255).

24. A detergent composition containing a polyphenol oxidase according to any one of claims 1 to 8.

# FIG. 1

# FIG. 2

# FIG. 3

# FIG. 4

# FIG. 5

# FIG. 6

FIG. 7

Activity peak

Elution volume （m l ）

FIG. 8

FIG. 9

# FIG. 10

# FIG. 11

# FIG. 12

# FIG. 13

FIG. 14

## INTERNATIONAL SEARCH REPORT

| | International application No. |
|---|---|
| | PCT/JP96/01594 |

| A. CLASSIFICATION OF SUBJECT MATTER |
|---|
| Int. Cl⁶ C12N9/02, C12N1/14, C11D3/386, D06L3/00 // (C12N9/02, C12R1:645) |
| According to International Patent Classification (IPC) or to both national classification and IPC |

| B. FIELDS SEARCHED |
|---|
| Minimum documentation searched (classification system followed by classification symbols) |
| Int. Cl⁶ C12N9/02, C12N1/14, C11D3/386, D06L3/00 |

Documentation searched other than minimum documentation to the extent that such documents are included in the fields searched

Electronic data base consulted during the international search (name of data base and, where practicable, search terms used)

BIOSIS PREVIEWS

## C. DOCUMENTS CONSIDERED TO BE RELEVANT

| Category* | Citation of document, with indication, where appropriate, of the relevant passages | Relevant to claim No. |
|---|---|---|
| A | JP, 6-316874, A (Kurabo Industries Ltd.), November 15, 1994 (15. 11. 94)(Family: none) | 1-10, 12 |
| A | JP, 62-278981, A (Takara Shuzo Co., Ltd.), December 3, 1987 (03. 12. 87)(Family: none) | 1-9, 14-23 |
| A | WO, 92/18687, A1 (NOVO NORDISK A/S), October 29, 1992 (29. 10. 92) & EP, 580707, A1 & JP, 6-506731, A & US, 5356437, A | 1-10, 12, 14-16 |
| A | WO, 93/13193, A1 (NOVO NORDISK A/S), July 8, 1993 (08. 07. 93) & EP, 617734, A1 & JP, 7-504694, A | 1-9, 11, 13-16, 24 |
| A | JP, 57-159487, A (Amano Pharmaceutical Co., Ltd.), October 1, 1982 (01. 10. 82)(Family: none) | 1-9, 17-21 |
| A | JP, 3-16698, A (Rinko Katayama), January 24, 1991 (24. 01. 91)(Family: none) | 1-10, 14-16 |

| [X] Further documents are listed in the continuation of Box C. | [ ] See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| September 4, 1996 (04. 09. 96) | September 17, 1996 (17. 09. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

| International application No. |
| --- |
| PCT/JP96/01594 |

**C (Continuation).  DOCUMENTS CONSIDERED TO BE RELEVANT**

| Category* | Citation of document, with indication, where appropriate, of the relevant passages | Relevant to claim No. |
| --- | --- | --- |
| A | US, 5091089, A (Development Center for Biotechnology), February 25, 1992 (25. 02. 92) (Family: none) | 1-10, 14-16 |
| A | Zeitschrift fuer Lebensmittel-Untersuchung und-Forschung, Vol. 169, No. 4 (1979), p. 271-275 | 1 - 8 |
| A | Phytochemistry, Vol. 20, No. 5 (1981), p. 1105-1112 | 1-8, 17-23 |
| A | Biochimica et Biophysica Acta, Vol. 1243, No. 1, (1995), p. 71-77 | 1 - 8 |

Form PCT/ISA/210 (continuation of second sheet) (July 1992)